# EUROPEAN PATENT APPLICATION

(11) **EP 4 620 461 A1**
(43) Date of publication of application: **24.09.2025**
(21) Application number: 24165421.9
(22) Date of filing: 22.03.2024
(51) Int. Cl.: A61K 31/00, A61K 31/167, A61K 31/277, A61K 31/4155, A61K 31/4166, A61K 31/4439, A61K 31/565, A61K 31/58, A61K 31/7105, A61K 38/00, A61K 39/00, A61K 45/06, A61P 35/00

(54) **TREATMENT OF PROSTATE CANCER BY INHIBITING SHORT PDE4D ISOFORMS**

(71) Applicant: Koninklijke Philips N.V., 5656 AG Eindhoven (NL)
(72) Inventor: HOFFMANN, Ralf Dieter, Eindhoven (NL)
(74) Representative: Philips Intellectual Property & Standards

(57) **Abstract**

The invention relates to a product for use in the treatment, prevention or amelioration of prostate cancer in a subject in need thereof, wherein the product reduces the expression of a short PDE4D isoform such as PDE4D1, PDE4D2 and/or PDE4D6. The product may for example be a dominant-negative form of a short PDE4D isoform a proteolysis targeting chimera specifically targeting a short PDE4D isoform or a small RNA for targeting short isoform PDE4D expression.

## Description

### FIELD OF THE INVENTION

The invention relates to a product for use in the treatment, prevention or amelioration of prostate cancer in a subject in need thereof, wherein the product reduces the expression of a short PDE4D isoform such as PDE4D1, PDE4D2 and/or PDE4D6. The product may for example be a dominant-negative form of a short PDE4D isoform a proteolysis targeting chimera specifically targeting a short PDE4D isoform or a small RNA for targeting short isoform PDE4D expression.

### BACKGROUND OF THE INVENTION

Cancer is a class of diseases in which a group of cells display uncontrolled growth, invasion and sometimes metastasis. These three malignant properties of cancers differentiate them from benign tumors, which are self-limited, do not invade or metastasize. Among men, the three most commonly diagnosed cancers are prostate, lung and colorectal cancer in developed countries. Particularly prostate cancer is the most common malignancy in European males. In 2002 in Europe, an estimated 225,000 men were newly diagnosed with prostate cancer and about 83,000 died from this disease.

Prostate cancer, for example, is traditionally diagnosed via the serum level of prostate-specific antigen (PSA). However, PSA is not prostate cancer-specific and can be raised in other circumstances, leading to a large number of false-positives (cancer is not found in around 70% of men with raised PSA levels who undergo biopsy). Furthermore, there will be an unpredictable number of false-negatives who later develop prostate cancer in the presence of a "normal" PSA test.

In patients who undergo treatment, the most important clinical prognostic indicators of disease outcome are the stage, pretherapy PSA level, and Gleason score. The higher the grade and the stage, the poorer the prognosis. Nomograms can be used to calculate the estimated risk of the individual patient. The predictions are based on the experience of large groups of patients. [250] A complicating factor is that the majority of patients have multiple independent tumor foci upon diagnosis, and these foci have independent genetic changes and molecular features. Because of this extensive inter-focal heterogeneity, it is a risk that the prognostication is set based on the wrong tumor focus.

Androgen ablation therapy causes remission in 80-90% of patients undergoing therapy, resulting in a median progression-free survival of 12 to 33 months. After remission, an androgen-independent phenotype typically emerges, wherein the median overall survival is 23-37 months from the time of initiation of androgen ablation therapy. How androgen-independence is established and how it reestablishes progression is unclear. Androgen ablation therapy may for example include taking antiandrogen drugs.

US 2012/129788 A1 relates to the 4D7 isoform of PDE and teaches that tumors have increased PDE4D7 expression, and this can be used for diagnosis purposes.

EP3434787 A1 teaches that expression of PDE4D7 is reduced in hormone resistance and diagnosis using PDE4D7 expression levels.

EP3303618 A1 relates to methods for determining gene expression signatures for diagnosing patients with prostate cancer, e.g., to establish a prognosis for such patients, for determining the predisposition of said patient to develop aggressive or indolent disease, for example after primary treatment, and/or for identification and stratification of patients for available therapies.

Henderson et al. (BRITISH JOURNAL OF CANCER, vol. 110, no. 5, 11 February 2014 (2014-02-11), pages 1278-1287) describes that PDE4D7 is downregulated in androgen-independent prostate cancer cells.

Boettcher et al. (ONCOTARGET, vol. 7, no. 43, 25 October 2016 (2016-10-25), pages 70669-70684) describes that PDE4D isoform composition is deregulated in primary prostate cancer and indicative for disease progression and development of distant metastases.

Therefore there is a constant need for new and improved methods for treatment of cancers such as prostate cancer.

These drawbacks, among others, are overcome by the products and methods as outlined in the appended claims.

### SUMMARY OF THE INVENTION

In a first aspect the invention relates to a composition for use in the treatment, prevention or amelioration of prostate cancer in a subject in need thereof, wherein the subject is an intermediate to high-risk prostate cancer patients with localized disease the use comprising administering to the subject the composition and an antiandrogen; wherein the composition comprises one or more agents for specifically reducing expression of one or more short isoforms of PDE4D selected from PDE4D1, PDE4D2 and PDE4D6.

In a second aspect the invention relates to an antiandrogen for use in the treatment, prevention or amelioration of prostate cancer in a subject in need thereof, wherein the subject is an intermediate to high-risk prostate cancer patients with localized disease the use comprising administering to the subject the composition and an antiandrogen; wherein the composition comprises one or more agents for specifically reducing expression of one or more short isoforms of PDE4D selected from PDE4D1, PDE4D2 and PDE4D6.

### BRIEF DESCRIPTION OF THE DRAWINGS

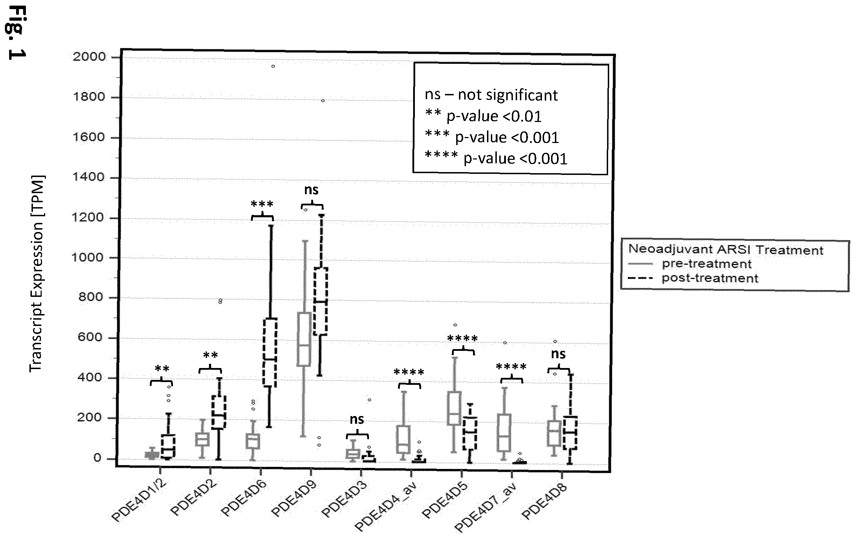

Fig. 1: depicts isoform specific expression levels pre- and post-antiandrogen treatment in a cohort of 35 patients with intermediate to high risk prostate cancer localized disease. All patients have an ISUP Gleason score between 2-5, with 80% a ISUP Gleason score of 4-5. Patients were treated for 6 months in anti androgens in a neo-adjuvant setting (ADT + enzalutamide). Biopsies were collected from prostate before onset of treatment with antiandrogen, at the end of treatment the prostate was resected by radical prostatectomy, and tissue punches were collected from the resected prostate tissue. Pre- and post-treatment tissues were subjected to RNAseq expression analysis, and RNA seq reads corresponding to specific isoforms of PDE4D are plotted. In grey are the pre- treatment expression levels and in black the post-treatment expression levels. PDE4D isoforms are grouped by short and long isoforms: the lefyt of the graph shows the short isoforms PDE4D1/2 (reads can belong to either PDE4D1 or PDE4D2), PDE4D2, PDE4D6, and PDE4D9. The right side of the graph depicts the long isoforms PDE4D3, PDE4D4, PDE4D5, PDE4D7 and PDE4D8. Significant changes in expression levels are indicated above the graphs, where ns indicates not significant, ** indicates a p value below 0.01., *** indicates a p value below 0.001 and **** indicates a p value below 0.0001.

### Definitions

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20%, preferably ±15%, more preferably ±10%, and even more preferably ±5%.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

"Antagonist" and "inhibitor": These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

"Anti-cancer effect": This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

"Alleviating cancer": The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

"Compositions", "products" or "combinations": These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

"Combination therapy", or "in combination with": These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

### DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention.
Unless otherwise defined, all technical and scientific terms used herein have the same meaning as commonly understood by a person of ordinary skill in the art.

Patients diagnosed with high risk localized prostate cancer have variable outcomes following surgery. Trials of intense neoadjuvant androgen deprivation therapy (NADT) have shown lower rates of recurrence among patients with minimal residual disease after treatment. The molecular features that distinguish exceptional responders from poor responders are not known.

The activation of G-protein coupled receptors and subsequent activation of adenyl cyclase catalyzes the conversion of ATP to cAMP, a second messenger involved in the regulation of numerous cellular processes. Phosphodiesterases (PDEs) are a superfamily of enzymes which play the fundamental role of hydrolysing cAMP, leading to its degradation and termination of cell signal transduction. These enzymes define and regulate intracellular cAMP gradients around specific signalling complexes, with each specific isoform having unique functional roles.

Of particular interest is the PDE4 family of enzymes, which is made up of over 25 different isoforms, many of which have important, non-redundant functions. Often, the function of a particular PDE4 isoform is conferred by its unique N-terminal, which acts as a "postcode" to anchor PDE4 enzymes to discrete intracellular domains where they sculpt signal-specific cAMP gradients. PDE4s also contain a catalytic unit and regulatory domains termed "upstream conserved regions one and two" (UCR1/2) which are highly conserved throughout the isoforms. All long-form PDE4s, such as for example PDE4D7, contain UCR1, which contains a PKA motif that becomes phosphorylated during conditions of raised cAMP. Such an action serves to activate PDE4 and rapidly reduce the local concentration of cAMP. This feedback loop underpins the transient nature of cAMP signals and ensures a rapid but fleeting response to activation of Gαs-coupled receptors.

Deregulated cAMP signalling is associated with various diseases including cancer, with the PDE4D sub-family of particular interest in prostate cancer (PCa). PDE4D7, a long PDE4D isoform variant, localises to the sub-plasma membrane in PCa cells and its expression inversely correlates with disease progression. Specifically, PDE4D7 mRNA is significantly downregulated from an androgen sensitive (AS) to androgen insensitive (AI) disease phenotype, implicating PDE4D7 in PCa development and progression. Furthermore, there is a significant association between PDE4D7 expression and presence of the TMPRSS2-ERG gene fusion, the most common gene rearrangement in PCa which leads to overexpression of ERG and subsequent enhanced PCa aggressiveness.

The different splice isoforms of PDE4D can be subdivided in short and long isoform, where short isoforms comprise essentially PDEase domain and no substantial N- or C terminal structures in the protein, whereas the long isoforms have long N-terminal structures besides a PDEase domain. It is observed that short isoform PDE4D proteins are predominantly found in the cytosol, while long isoforms are predominately membrane bound or located near or at cellular structures. It thus appears that the N-terminal part of the long isoform PDE4Ds mediate distinct cellular localization and consequently it is likely that the long and short isoforms have distinct physiological functions. Examples of short isoforms are PDE4D1, PDE4D2, and PDE4D6. Examples of long isoforms are PDE4D4, PDE4D5, and PDE4D7.

Herein the inventors describe data obtained from intermediate to high risk cancer patients who have received anti-androgens in a neoadjuvant setting. For these patients sequencing data was obtained from tumor biopsies pre- and post-antiandrogen treatment. From these sequencing data, isoform specific sequencing reads were isolated to obtain expression information on the specific isoforms and compare pre- and post-antiandrogen treatment expression levels in the tumor. These results are presented in Figure 1. Strikingly, PDE4D1, PDE4D2, and PDE4D6, all short isoforms, are significantly upregulated in tumors post-antiandrogen treatment, while PDE4D4, PDE4D5, and PDE4D7, all long isoforms, are significantly downregulated in the tumor post-antiandrogen treatment.

Downregulation of PDE4D7 in response to treatment has been described in prostate cancer, and upregulating PDE4D7 has been suggested as a supplementary treatment to conventional treatment such as antiandrogens. The data presented here however makes it plausible that reducing expression of short isoforms of PDE4D, particularly PDE4D1, PDE4D2, or PDE4D6, may provide an alternative or complementary treatment strategy.

Thus the experimental results described herein support that the effectiveness of antiandrogen therapy in prostate cancer may be enhanced by downregulating short PDE4D isoforms, such as PDE4D1, PDE4D2 or PDE4D6.

Therefore in a first aspect the invention relates to a composition for use in the treatment, prevention or amelioration of prostate cancer in a subject in need thereof, wherein the subject is an intermediate to high-risk prostate cancer patient with localized disease the use comprising administering to the subject the composition and an antiandrogen; wherein the composition comprises one or more agents for specifically reducing expression of one or more short isoforms of PDE4D selected from PDE4D1, PDE4D2 and PDE4D6.

In a further embodiment the invention relates to a method of treating, preventing or ameliorating prostate cancer in a subject in need thereof, the method comprising administering to the subject the composition and an antiandrogen; wherein the composition comprises one or more agents for specifically reducing expression of one or more short isoforms of PDE4D selected from PDE4D1, PDE4D2 and PDE4D6, and wherein the subject is an intermediate to high-risk prostate cancer patient with localized disease.

Further disclosed herein is a therapy for use in the treatment, prevention or amelioration of prostate cancer in a subject in need thereof, wherein the subject is an intermediate to high-risk prostate cancer patients with localized disease, the therapy comprising administering to the subject a product and an antiandrogen, wherein the product is a product for specifically reducing expression of one or more short isoforms of PDE4D selected from PDE4D1, PDE4D2 and PDE4D6.

Further disclosed herein is a method of treating, preventing or ameliorating prostate cancer in a subject in need thereof, wherein the subject is an intermediate to high-risk prostate cancer patients with localized disease, the method of treatment comprising administering to the subject a product and an antiandrogen; wherein the product is a product for specifically reducing expression of one or more short isoforms of PDE4D selected from PDE4D1, PDE4D2 and PDE4D6.

In a second aspect the invention relates to an antiandrogen for use in the treatment, prevention or amelioration of prostate cancer in a subject in need thereof, wherein the subject is an intermediate to high-risk prostate cancer patients with localized disease the use comprising administering to the subject the composition and an antiandrogen; wherein the composition comprises one or more agents for specifically reducing expression of one or more short isoforms of PDE4D selected from PDE4D1, PDE4D2 and PDE4D6.

Details, examples and explanations of the main aspects and disclosures of the invention are provided below.

It is hypothesized that reducing expression of one or more of the short isoforms PDE4D1, PDE4D2 and PDE4D6, when combined with antiandrogen treatment further reduces tumor burden and so reduces chances of recurrence after removal of the tumor and/or prostate, e.g. by radical prostatectomy or radiotherapy. It is speculated that the proposed combination therapy is particularly useful for patients in the high risk group. Therefore, in an embodiment the subject has an International Society for Urological Pathology (ISUP) score of 2-5, preferably 4 or 5. Alternatively the patient has a Gleason score of 7-10. An ISUP score of 2-3 corresponds to a Gleason score of 7 (3+4 or 4+3) and refers to intermediate risk prostate cancer patients. An ISUP score of 4-5 corresponds to a Gleason score of 8-10 and corresponds to high or very high risk prostate cancer patients.

Antiandrogen therapies suitable for the treatment of prostate cancer are known to the skilled person. Non limiting examples include luteinizing hormone releasing hormone (LHRH) agonists, LHRH antagonists (also referred to as GnRH antagonists), estrogens, androgen receptor blockers, androgen synthesis inhibitors. Non limiting examples of LHRH agonists include leuprolide (Lupron), goserelin (Zoladex), triptorelin (Trelstar), and histrelin (Vantas). Non limiting examples of LHRH antagonists include degarelix (Firmagon) and relugolix (Orgovyx). Non limiting examples of androgen receptor blockers include first generation drugs such as flutamide, bicalutamide (Casodex), and nilutamide (Nilandron), and second-generation drugs such as enzalutamide (Xtandi), apalutamide (Erleada), and darolutamide (Nubeqa). Non limiting examples of androgen synthesis inhibitors include abiraterone (Yonsa, Zyriga), ketoconazole, and aminoglutethimide. Thus in an embodiment the antiandrogen is selected from LHRH agonists, LHRH antagonists, estrogens, androgen receptor blockers, and androgen synthesis inhibitors. In an embodiment the antiandrogen is selected from degarelix (Firmagon), relugolix (Orgovyx), flutamide, bicalutamide (Casodex), and nilutamide (Nilandron), enzalutamide (Xtandi), apalutamide (Erleada), darolutamide (Nubeqa), abiraterone (Yonsa, Zyriga), ketoconazole, and aminoglutethimide. In a preferred embodiment the antiandrogen is a first or second generation androgen receptor blocker. In a preferred embodiment the antiandrogen is selected from flutamide, bicalutamide (Casodex), and nilutamide (Nilandron), enzalutamide (Xtandi), apalutamide (Erleada), and darolutamide (Nubeqa). In a more preferred embodiment the antiandrogen is a second generation androgen receptor blocker. In an embodiment the antiandrogen is selected from enzalutamide (Xtandi), apalutamide (Erleada), and darolutamide (Nubeqa). In a further embodiment the anitandorgen is selected from abiraterone acetate, enzalutamide, apalutamide, or darolutamide, flutamide, bicalutamide, nilutamide and estrogens.

Although the data presented here is obtained from patients receiving antiandrogen treatment in a neoadjuvant setting, it is anticipated that any type of antiandrogen treatment may benefit from reducing expression of short isoform PDE4D as broadly described herein. Thus in an embodiment the antiandrogen is provided in a neoadjuvant, adjuvant or salvage setting. In a preferred embodiment the antiandrogen is provided in a neoadjuvant setting.

Neoadjuvant therapy is the administration of therapeutic agents before a main treatment. In treatment of prostate cancer neoadjuvant androgen deprivation therapy usually refers to a treatment regiment with antiandrogens prior to surgery such as radical prostatectomy or radiation therapy such as radical radiotherapy. Adjuvant therapy, also known as adjunct therapy, adjuvant care, or augmentation therapy, is a therapy that is given in addition to the primary or initial therapy to maximize its effectiveness. Thus neoadjuvant therapy differs from adjuvant therapy in that neoadjuvant therapy is provided prior to the main treatment. Neoadjuvant is typically used to reduce the tumor size before the main treatment,

The present invention aims to reduce short PDE4D isoform expression levels, specifically PDE4D1, PDE4D2, and/or PDE4D6 expression, thereby reducing the amount of PDE4D1, PDE4D2, and/or PDE4D6 protein present in the prostate cancer cells. Without wishing to be bound by theory, it is hypothesize that such reduction in protein in the prostate cancer cells prevents the tumor cells from growing resistant to antiandrogen therapy, or alternatively being more susceptible to the effects of such antiandrogen therapy. Adjuvant therapy for prostate cancer is typically provided after surgery to improve the patient outcome. Salvage therapy, also known as rescue therapy, is a form of therapy given after the cancer has not responded to other cancer treatment regimens.

The term "phosphodiesterase 4D1" or "PDE4D1" relates to the splice variant 1 of the human phosphodiesterase PDE4D, i.e. the human phosphodiesterase PDE4D1 gene, preferably to the sequence as defined in NCBI Reference Sequence: NM_001197222.1, more preferably to the nucleotide sequence as set forth in SEQ ID NO: 1, which corresponds to the sequence of the above indicated NCBI Reference Sequence of the PDE4D1 transcript, and also relates to the corresponding amino acid sequence as set forth in SEQ ID NO: 2, which corresponds to the protein sequence defined in NCBI Protein Accession Reference Sequence NP _001184151.1 encoding the PDE4D1 polypeptide. The term "phosphodiesterase 4D1" or "PDE4D1" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D2" or "PDE4D2" relates to the splice variant 2 of the human phosphodiesterase PDE4D, i.e. the human phosphodiesterase PDE4D2 gene, preferably to the sequence as defined in NCBI Reference Sequence: NM_001197221.1, more preferably to the nucleotide sequence as set forth in SEQ ID NO: 3, which corresponds to the sequence of the above indicated NCBI Reference Sequence of the PDE4D2 transcript, and also relates to the corresponding amino acid sequence as set forth in SEQ ID NO: 4, which corresponds to the protein sequence defined in NCBI Protein Accession Reference Sequence NP _001184150.1 encoding the PDE4D2 polypeptide. The term "phosphodiesterase 4D2" or "PDE4D2" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D3" or "PDE4D3" relates to the splice variant 3 of the human phosphodiesterase PDE4D, i.e. the human phosphodiesterase PDE4D3 gene, preferably to the sequence as defined in NCBI Reference Sequence: NM_006203.4, more preferably to the nucleotide sequence as set forth in SEQ ID NO: 5, which corresponds to the sequence of the above indicated NCBI Reference Sequence of the PDE4D3 transcript, and also relates to the corresponding amino acid sequence as set forth in SEQ ID NO: 6, which corresponds to the protein sequence defined in NCBI Protein Accession Reference Sequence NP_006194.2 encoding the PDE4D3 polypeptide.

The term "phosphodiesterase 4D4" or "PDE4D4" relates to the splice variant 4 of the human phosphodiesterase PDE4D, i.e. the human phosphodiesterase PDE4D4 gene, preferably to the sequence as defined in NCBI Reference Sequence: NM_001104631.1, more preferably to the nucleotide sequence as set forth in SEQ ID NO: 7, which corresponds to the sequence of the above indicated NCBI Reference Sequence of the PDE4D4 transcript, and also relates to the corresponding amino acid sequence as set forth in SEQ ID NO: 8, which corresponds to the protein sequence defined in NCBI Protein Accession Reference Sequence NP_001098101.1 encoding the PDE4D4 polypeptide.

The term "phosphodiesterase 4D5" or "PDE4D5" relates to the splice variant 5 of the human phosphodiesterase PDE4D, i.e. the human phosphodiesterase PDE4D5 gene, preferably to the sequence as defined in NCBI Reference Sequence: NM_001197218.1, more preferably to the nucleotide sequence as set forth in SEQ ID NO: 9, which corresponds to the sequence of the above indicated NCBI Reference Sequence of the PDE4D5 transcript, and also relates to the corresponding amino acid sequence as set forth in SEQ ID NO: 10, which corresponds to the protein sequence defined in NCBI Protein Accession Reference Sequence NP _001184147.1 encoding the PDE4D5 polypeptide. The term "phosphodiesterase 4D5" or "PDE4D5" also relates to the amplicon that can be generated by the primer pair PDE1D5_forward (SEQ ID NO: 23) and the PDE1D5_reverse (SEQ ID NO: 24) and can be detected by probe SEQ ID NO: 25.

The term "phosphodiesterase 4D6" or "PDE4D6" relates to the splice variant 6 of the human phosphodiesterase PDE4D, i.e. the human phosphodiesterase PDE4D6 gene, preferably to the sequence as defined in NCBI Reference Sequence: NM_001197223.1, more preferably to the nucleotide sequence as set forth in SEQ ID NO: 11, which corresponds to the sequence of the above indicated NCBI Reference Sequence of the PDE4D6 transcript, and also relates to the corresponding amino acid sequence as set forth in SEQ ID NO: 12, which corresponds to the protein sequence defined in NCBI Protein Accession Reference Sequence NP _001184152.1 encoding the PDE4D6 polypeptide.

The term "phosphodiesterase 4D7" or "PDE4D7" relates to the splice variant 7 of the human phosphodiesterase PDE4D, i.e. the human phosphodiesterase PDE4D7 gene, preferably to the sequence as defined in NCBI Reference Sequence: NM_001165899.1, more preferably to the nucleotide sequence as set forth in SEQ ID NO: 13, which corresponds to the sequence of the above indicated NCBI Reference Sequence of the PDE4D7 transcript, and also relates to the corresponding amino acid sequence as set forth in SEQ ID NO: 14, which corresponds to the protein sequence defined in NCBI Protein Accession Reference Sequence NP_001159371.1 encoding the PDE4D7 polypeptide. The term "phosphodiesterase 4D7" or "PDE4D7" also relates to the amplicon that can be generated by the primer pair PDE1D7_forward (SEQ ID NO: 26) and the PDE1D7_reverse (SEQ ID NO: 27) and can be detected by probe SEQ ID NO: 28.

The term "phosphodiesterase 4D8" or "PDE4D8" relates to the splice variant 8 of the human phosphodiesterase PDE4D, i.e. the human phosphodiesterase PDE4D8 gene, preferably to the sequence as defined in NCBI Reference Sequence: NM_001197219.1, more preferably to the nucleotide sequence as set forth in SEQ ID NO: 15, which corresponds to the sequence of the above indicated NCBI Reference Sequence of the PDE4D8 transcript, and also relates to the corresponding amino acid sequence as set forth in SEQ ID NO: 16, which corresponds to the protein sequence defined in NCBI Protein Accession Reference Sequence NP _001184148.1 encoding the PDE4D8 polypeptide.

The term "phosphodiesterase 4D9" or "PDE4D9" relates to the splice variant 9 of the human phosphodiesterase PDE4D, i.e. the human phosphodiesterase PDE4D9 gene, preferably to the sequence as defined in NCBI Reference Sequence: NM_001197220.1, more preferably to the nucleotide sequence as set forth in SEQ ID NO: 17, which corresponds to the sequence of the above indicated NCBI Reference Sequence of the PDE4D9 transcript, and also relates to the corresponding amino acid sequence as set forth in SEQ ID NO: 18, which corresponds to the protein sequence defined in NCBI Protein Accession Reference Sequence NP _001184149.1 encoding the PDE4D9 polypeptide. The term "phosphodiesterase 4D9" or "PDE4D9" also relates to the amplicon that can be generated by the primer pair PDE1D5_forward (SEQ ID NO: 29) and the PDE1D5_reverse (SEQ ID NO: 30) and can be detected by probe SEQ ID NO: 31.

The terms "PDE4D1", "PDE4D2", "PDE4D3", "PDE4D4", "PDE4D5", "PDE4D6", "PDE4D7", "PDE4D8" and "PDE4D9" also comprises nucleotide sequences showing a high degree of homology to PDE4D1, PDE4D2, PDE4D3, PDE4D4, PDE4D5, PDE4D6, PDE4D7, PDE4D8 and PDE4D9 respectively, e.g. nucleic acid sequences being at least 75%, 80%, 85%, 90%, 91%, 92%, 93%, 94%, 95%, 96%, 97%, 98% or 99% identical to the sequence as set forth in SEQ ID NOs: 1, 3, 5, 7, 9, 11, 13, 15 or 17 respectively or amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 respectively or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1, 3, 5, 7, 9, 11, 13, 15 or 17.

The term "expression level" as used herein refers to the amount of PDE4D variant transcript and/or PDE4D protein derivable from a defined number of cells or a defined tissue portion, preferably to the amount of PDE4D variant transcript and/or PDE4D variant protein obtainable in a standard nucleic acid (e.g. RNA) or protein extraction procedure. Suitable extraction methods are known to the person skilled in the art.

The present invention is prompted by the inventors finding that the short isoform PDE4D genes PDE4D1, PDE4D2 and PDE4D6 have increased expression post antiandrogen treatment in prostate cancer patients undergoing neoadjuvant androgen deprivation therapy. The data make plausible that reducing expression of PDE4D1, PDE4D2 and/or PDE4D6 provides an a supplement to antiandrogen therapy which may render the antiandrogen therapy more effective, as it is postulated that increased expression of PDE4D1, PDE4D2 and PDE4D6 provides a mechanism of tumor resistance to antiandrogen therapy. Thus reducing expression of PDE4D1, PDE4D2 and/or PDE4D6 in prostate cancer cells may have several beneficial effects such as reduction of cancer cell proliferation and reversal of therapy resistance. It is envisaged that such effect can thus be established by decreasing the expression of PDE4D1, PDE4D2 and/or PDE4D6 protein, that is, by decreasing the amount of PDE4D1, PDE4D2 and/or PDE4D6 protein in the prostate cancer cells or indirectly by decreasing the expression level(s) of the PDE4D1, PDE4D2 and/or PDE4D6 isoform(s) of the PDE4D gene, that is, by reducing transcription of the PDE4D1, PDE4D2 and/or PDE4D6 isoform(s) or by otherwise decreasing the amount of PDE4D1, PDE4D2 and/or PDE4D6 mRNA.

The methods and uses disclosed herein preferably result in an improvement or cure of the subject. The improvement of the subject may for example refer to a reduced tumor size, reduced tumor burden, reduced chance of tumor metastasis, improved average overall survival time or reduced time to overall death or cancer specific death.

When used herein *tumor size* refers to the volume of a tumor at a predetermined time point after treatment. When used herein, *tumor burden* refers to the number of cancer cells, the size of the tumor or number of metastasis at a predetermined time point after treatment. When used herein *chance of tumor metastasis* refers to the chance that one or more metastases will develop from the prostate cancer at a predetermined time point after treatment. When used herein number of positive lymph nodes refers the number of lymph nodes in the prostate cancer subject where at least one tumor cell can be identified. Preferably the cancer cell is a cancer cell derived from the prostate cancer. The positive lymph nodes may be local or peripheral. When used herein, probability of survival refers to the chance that the prostate cancer subject is still alive at a predetermined time point after treatment. When used herein the term *overall survival* refers to the (average) length of survival form the date of taking the biological sample from the subject. Cancer free survival refers to the (average) time the subject live without detectable cancer after the date of taking the biological sample from the subject. When used herein the term *overall death* refers to the (average) length until death from the date of taking the biological sample from the subject. When used herein the term *cancer specific death* refers to the (average) length until death caused as a result of the prostate cancer (or a metastasis thereof) from the date of taking the biological sample from the subject.

Therefore, the present disclosure broadly defines a composition for reducing the expression of PDE4D1, PDE4D2 and/or PDE4D6. In an aspect the composition is a compound or combination of compounds or a product.

When used herein the term compound may refer to a chemical compound (also referred to as "small molecule therapy"), a biological such but not limited to a polynucleotide, a polypeptide, or a sugar polymer.

When used herein the term composition may refer to a mixture of compounds or a combination of a compound with adjuvants. A composition may be a solution or a dry composition. The term composition further refers to a combination therapy, for example of two or more different agent aimed to reduce expression of a short isoform PDE4D or two or combination of agents aiming to increase expression of a long isoform PDE4D and reduce expression of a short isoform PDE4D. The different agents comprised in the composition may be administered simultaneously or sequentially.

The inventors envision several ways to achieve decreased PDE4D1, PDE4D2 and/or PDE4D6 expression in a subject, some non-limiting examples are listed below, however it is understood that the disclosure extends to any method or compound known to the skilled person to achieve decreased expression of PDE4D1, PDE4D2 and/or PDE4D6 isoform(s). Thus in an aspect of the disclosure the composition comprises one or more agents that allows for the expression in the prostate tumor of the subject selected from 1) an agent for expressing dominant negative form of a short isoform PDE4D, 2) a proteolysis targeting chimera agent specifically targeting a shorth isoform PDE4D, or 3) an RNA agent for specifically inhibiting the expression of a short isoform PDE4D. Thus in an embodiment the product comprises one or more agents that allow for the expression in the prostate tumor of the subject selected from: - an agent for expressing a dominant-negative form of PDE4D1 (Asp396Ala), PDE4D2 (Asp318Ala), and/or PDE4D6 (Asp329Ala); - a proteolysis targeting chimera (PROTAC) agent specifically targeting PDE4D1, PDE4D2, and/or PDE4D6; and - an RNA agent for specifically inhibiting the expression of PDE4D1, PDE4D2 and/or PDE4D6.

In an embodiment the agent as envisioned herein is a gene therapy. Thus in an embodiment the composition as defined herein is for providing a gene therapy to express one or more of a dominant-negative form of PDE4D1 (Asp396Ala), PDE4D2 (Asp318Ala), and/or PDE4D6 (Asp329Ala); a proteolysis targeting chimera (PROTAC) specifically targeting PDE4D1, PDE4D2, and/or PDE4D6; and a small RNA for specifically inhibiting the expression of PDE4D1, PDE4D2 and/or PDE4D6.

When used herein, a gene therapy refers to a method to introduce a nucleotide in a cell of a subject, preferably a tumor cell such as a prostate tumor cell. The nucleotide may for example be a viral vector or a non-viral vector, however it is understood that any nucleotide which can be introduced in a cell of a patient and express a protein based agent or an RNA agent as broadly described herein may be used. The term gene therapy also comprises introducing a nucleotide in a cell ex vivo or in vitro and introducing the modified cell in the subject.

Viral vectors are a known strategy for cell transformation, typically viral-vector based cell transformation use modified viruses as vehicles to introduce specific DNA or RNA sequences into cells. The vector is packaged using the viral proteins allowing infection of cells and expression of its viral genome. Typically, the viral vector is modified such that no new viral particles can be produced upon infection of the target cell. Non limiting examples include retroviruses (RV), adenoviruses (AV), adeno-associated viruses (AAV), lentiviruses (LV), and herpes simplex viruses (HSV). Other ways to use viral particles or viral vectors to introduce the encoding nucleotide in a cell such as a tumor cell are known to the person skilled in the art and also envisaged to be encompassed by the invention.

Alternatively, to viral vector-based cell transformation, a non-viral vector may be used. These vectors typically contain a promotor to drive expression of the relevant construct (e.g., a dominant negative form of a short isoform PDE4D, a PROTAC agent or an RNA agent), and typically require some means to introduce the vector into the target cell. Means to deliver a non-viral vector to a target cell are known to the skilled person, and for example reviewed in Ramamoorth M, Narvekar A. Non-viral vectors in gene therapy- an overview. J Clin Diagn Res. 2015 Jan;9(1):GE01-6 (hereby incorporated by reference in its entirety). For example, nanoparticles can be used to encapsulate the vector. Non limiting examples are lipid-based nanoparticles, peptide based nanoparticles, cationic lipid based nanoparticles, apolipoprotein based nanoparticles, (synthetic) polymer based nanoparticles such as polyethylenimine (PEI), chitosan, polylactate, polylactide, polyglucoside, denriomer or polymethracylate based nanoparticles. When used herein a nanoparticle is a small particle which can be used as a carrier to deliver a cargo (payload) in a patient. Preferably the cargo is a nucleotide encoding an agent as broadly defined herein above. Therefore, a nanoparticle can be used to deliver the cargo that induces the expression of the agent in a cell, e.g. a tumor cell or a prostate cancer cell. Other ways to use non-viral vectors to introduce the agent encoding nucleotide are known to the person skilled in the art and also envisaged to be encompassed by the invention.

Alternatively, to viral or non-viral vector-based cell transformation, a mRNA based approach may be used. This approach typically comprises the delivery of coding mRNA molecule (e.g. encoding a dominant negative form of a short isofrm PDE4D, a PROTAC,or a small RNA). The mRNA might be modified by methods known in the field to stabilize the RNA *in vivo* and increase its half-life (e.g., use of chemically modified nucleotides, codon optimization, optimization 5'-capping and 3'-tailing). Further, the mRNA may contain structures to optimize the 5'-UTR for increased mRNA translation or elements that allow the self-amplification of the mRNA. Means to deliver the mRNA into a target cell are known to the skilled person, and for example reviewed in Rohner E et al. Unlocking the promise of mRNA therapeutics. Nature Biotechnology 2022, 40, 1586-1600 (hereby incorporated by reference in its entirety). For example, lipid nanoparticles (LNPs) can be used to encapsulate the mRNA. Alternatively, extracellular vesicles (EV), cells, or biomimetics can be used to deliver the cargo that induces the expression of the agent to a cell, e.g. a tumor cell or a prostate cancer cell.

Therefore, in an embodiment the agent is delivered by a cell transformation method, preferably the cell transformation method is selected from: a viral vector capable of expressing the agent in a cell or a non-viral vector capable of expressing the agent in a cell. In an embodiment the agent or agent encoding nucleotide is delivered to the tumor cell using a nanoparticle.

When used herein the term vector is used to indicate any particle (e.g., a plasmid, a cosmid, a Lambda phage) used as a vehicle to artificially carry a foreign nucleic acid molecule - usually DNA - into another cell, where it can be replicated and/or expressed.

Dominant negative forms of are known to the skilled person and for example have been described in McCahill et al. Cell Signal. 2005 Sep;17(9):1158-73 (hereby incorporated by reference in its entirety). Typically dominant negative mutations comprise of point mutations of essential amino acids in the catalytic site, in the present case the PDEase domain. Typically dominant negative forms are catalytically not active and have a dominant negative function by competing with wildtype (catalytically active) protein by competing for their substrate, in the present case cyclic AMP. Exemplary dominant negative forms of short isoform PDE4Ds are PDE4D1 Asp396Ala, PDE4D2(Asp318Ala, and PDE4D6 Asp329Ala, in all of which an asparagine in the active site is mutated to alanine. It is however understood that other dominant negative forms of these isoforms exist and the invention should thus not be limited to the examples provided here. The skilled person is aware how to identify or design other dominant negative mutations of short isofrm PDE4Ds. For example simply scanning the protein sequence in a tool such as Prosite (https://prosite.expasy.org/) allows for the identification of the PDEase catalytic domain and essential amino acids in the site responsible for catalysis and binding of the substrate. Thus mutating one of the essential amino acids as identified by such tool is likely to result in a dominant negative form of the protein.

Proteolysis targeting chimeras (PROTACs) are chimeric proteins comprising of a targeting subunit and a protease targeting subunit for E3 targeting. The technology is for example described in Liu et al. Mol Biomed. 2022 Dec; 3: 46., hereby incorporated by reference in its entirety. Briefly summarized that targeting subunit of the PROTAC binds the target of interest and the protease subunit of the PROTAC targets the PROTAC/target complex to the E3 complex to be ubiquitinated and subsequent degradation by the proteasome. The technology allows for the specific degradation of proteins of intertest inside a cell by expressing the PROTAC.

It is envisioned that this technology can be used to reduce protein expression of PDE4D1, PDE4D2, and/or PDE4D6. Specific targeting of the PDE4D1, PDE4D2, and/or PDE4D6 isoforms may for example be achieved by an antibody or antigen binding fragment of an antibody, a targeting peptide, a small molecule or an aptamer. For example the antibody or antigen binding fragment of an antibody may be a small antigen binding protein such as but not limited to a single domain antibody (nanobody), a VHH fragment, a FAB fragment, a heavy chain antibody or a scFv antibody. Thus in an embodiment the PROTAC comprises a E3 targeting subunit and a targeting unit selected from n antibody or antigen binding fragment of an antibody, a targeting peptide, a small molecule or an aptamer, such as an antibody or antigen binding fragment is selected from a single domain antibody (nanobody), a VHH fragment, a FAB fragment, a heavy chain antibody or a scFv antibody, wherein the targeting unit specifically binds to the PDE4D1, PDE4D2, and/or PDE4D6 isoforms of PDE4D. A targeting peptide when used herein refers to a peptide specifically binding to a target, such as a short isoform PDE4D. A targeting peptide may for example mimic (part of) a substrate of its intended target or mimic (part of) an interacting protein of its intended target. When used herein a small molecule refers to a small molecule capable of binding a target protein, such as a small isoform PDE4D. For example the small molecule may specifically interact with the catalytic site of the protein for example by mimicking its substrate (cyclic AMP or AMP). When used herein an aptamer is a nuclei acid molecule, such as a DNA or an RNA molecule, which can form secondary structures and specifically bind to a target such as a short isoform PDE4D equivalent to antibody boding to an antigen.

RNA agents may be used to reduce expression of a specific gene or splice variant by specifically targeting the gene, as for example described in Pushparaj et al.. J Dent Res. 2008 Nov;87(11):992-1003, herewith incorporated by reference in its entirety. For example siRNA or miRNA technology may be used to specifically target the PDE4D1, PDE4D2, and/or PDE4D6 expression. Alternatively the RNA agent may be an oligonucleotide targeting an intron-exon or exon-intron boundary of PDE4D1, PDE4D2, and/or PDE4D6 thereby preventing or reducing expression, or producing a non-functional protein. This may be achieved for example by expressing a vector encoding a shRNA precursor of a miRNA or siRNA precursor or by directly introducing the siRNA (precursor), shRNA or miRNA into the cell.

Therapeutic approaches based on siRNA involve the introduction of a synthetic siRNA into the target cells to elicit RNA interference (RNAi), thereby inhibiting the expression of a specific messenger RNA (mRNA) to produce a gene silencing effect. By contrast, miRNA-based therapeutics may be based on miRNA replacement. In the replacement approach, synthetic miRNAs (also known as miRNA mimics) are used to mimic the function of the endogenous miRNAs. It thus leads to mRNA degradation/inhibition, and produces a gene silencing effect.

It is understood that the RNA agent may also be a synthetic oligonucleotide such as but not limited to phosphorothioate (PS) bond modified nucleotides and 2'-ribose modifications such as 2'-O-methyl (2'OMe) or 2'-O-methoxyethyl (2'MOE).

It is further envisioned that reducing expression of a short PDE4D isoform as described herein is complemented with increasing the expression of a long PDE4D isoform, such as PDE4D4, PDE4D5 and/or PDE4D7. Thus in an embodiment the product further comprises an agent that allows for the expression in the prostate tumor of the subject one or more of:
- PDE4D4, PDE4D5 and/or PDE4D7 or a mutationally activated form thereof;
- A deubiquitinase-targeting chimera (DUBTAC) for specifically preventing the degradation of PDE4D4, PDE4D5, and/or PDE4D7.

Similarly as dominant negative mutants for short isoforms, activated forms of long isoforms can be selected for or designed and expressed in the tumor cell. A non-limiting example is PDE4D7 Ser42Ala which is constitutively active, see e.g. Byrne et al., FEBS Lett. 2015 Mar 12;589(6):750-5 (incorporated by reference in its entirety).

Deubiquitinase targeting chimeras (DUBTACs) are chimeric proteins comprising of a targeting subunit and a deubiquitinase targeting subunit for targeting a deubiquitinase protein. The technology is for example described in Omar et al. 116 (27) 13320-13329, hereby incorporated by reference in its entirety. Briefly summarized the targeting subunit of the DUBTAC binds the target of interest and the deubiquitinase subunit of the DUBTAC targets the PROTAC/target complex to a deubiquitinase enzyme which protects the complex from being ubiquitinated and subsequent degradation by the proteasome, thus essentially preventing destruction of the protein of interest. The technology allows for the specific upregulation of proteins of intertest (by preventing degradation) inside a cell by expressing the DUBTAC.

It is envisioned that this technology can be used to increase protein expression of PDE4D4, PDE4D5, and/or PDE4D7. Specific targeting of the PDE4D4, PDE4D5, and/or PDE4D7 isoforms may for example be achieved by an antibody or antigen binding antibody fragment, a targeting peptide, a small molecule or an aptamer. The antibody or antigen binding antibody fragment may for example be a small antigen binding protein such as but not limited to a single domain antibody (nanobody), a VHH fragment, a FAB fragment, a heavy chain antibody or a scFv antibody. Thus in an embodiment the DUBTAC comprises a deubiquitinase targeting subunit and a targeting unit selected from an antibody or antibody fragment, a targeting peptide, a small molecule or an aptamer, such as a single domain antibody (nanobody), a VHH fragment, a FAB fragment, a heavy chain antibody or a scFv antibody, wherein the targeting unit specifically binds to the PDE4D4, PDE4D5, and/or PDE4D7 isoforms of PDE4D.

Thus in an embodiment the PROTAC and/or DUBTAC specifically targets PDE4D1, PDE4D2, and/or PDE4D6, and/or PDE4D4, PDE4D5, and/or PDE4D7 with a PDE4D isotype specific targeting unit selected from: an antibody or antibody fragment, a targeting peptide, a small molecule or an aptamer. In an embodiment the an antibody or antibody fragment is selected from a single domain antibody (nanobody), a VHH fragment, a FAB fragment, a heavy chain antibody or a scFv antibody.

Depending on the mode of introducing the agent in the tumor cells it is envisioned that either a single administration of the agent suffices or that repeated administration is required. For example when the selected mode of introducing the agent is using a viral vector (e.g. AAV based therapy) it is expected that such therapy results in stable expression of the agent in cells, and likely a single or a few (e.g. 1, 2, 3, 4 or 5) administrations of the agent to the patient suffices. Alternatively when introducing protein or RNA through nanoparticles transient expression of the agent is to be expected and the agent may be administered on a repeated basis, e.g. daily, weekly, biweekly, or monthly. Therefore, in an embodiment the product is administered prior to the antiandrogen, concurrent with the antiandrogen or after the antiandrogen. In an embodiment the antiandrogen is provided for 3 to 12 months, preferably between 4 and 10 months more preferably between 5 and 8 months. In an embodiment the therapy further comprises locoregional therapy, preferably radical prostatectomy (RP) or radiation therapy after conclusion of the antiandrogen treatment.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

### Examples

Having now generally described the invention, the same will be more readily understood through reference to the following examples which is provided by way of illustration and is not intended to be limiting of the present invention.

### Example 1

Isoform specific expression levels were determined pre- and post-antiandrogen treatment in a cohort of 35 patients with intermediate to high risk prostate cancer localized disease. All patients have an ISUP Gleason score between 2-5, with 80% a ISUP Gleason score of 4-5. Patients were treated for 6 months in anti androgens in a neo-adjuvant setting (ADT + enzalutamide). Biopsies were collected from prostate before onset of treatment with antiandrogen, at the end of treatment the prostate was resected by radical prostatectomy, and tissue punches were collected from the resected prostate tissue. Pre- and post-treatment tissues were subjected to RNAseq expression analysis, and RNA seq reads corresponding to specific isoforms of PDE4D are plotted. In grey are the pre- treatment expression levels and in black the post-treatment expression levels. PDE4D isoforms are grouped by short and long isoforms: the lefyt of the graph shows the short isoforms PDE4D1/2 (reads can belong to either PDE4D1 or PDE4D2), PDE4D2, PDE4D6, and PDE4D9. The right side of the graph depicts the long isoforms PDE4D3, PDE4D4, PDE4D5, PDE4D7 and PDE4D8. Significant changes in expression levels are indicated above the graphs, where ns indicates not significant, ** indicates a p value below 0.01., *** indicates a p value below 0.001 and **** indicates a p value below 0.0001. The results are depicted in Figure 1.

The expression of the PDE4D isoforms were measured by the quantification of the reads that map to the transcript specific regions of the respective isoform. The quantification was done for the sample before neoadjuvant ARSI (Androgen signaling inhibition) treatment was started and again (for the same patient) in a sample collected after prostate resection by radical prostatectomy. Shown is the PDE4D transcript expression in the pre- vs the post-treatment samples as a boxplot. The p-values of the expression difference between the pre- and post-treatment samples are indicated.

## Claims

1. A composition for use in the treatment, prevention or amelioration of prostate cancer in a subject in need thereof, wherein the subject is an intermediate to high-risk prostate cancer patients with localized disease
the use comprising administering to the subject the composition and an antiandrogen;
wherein the composition comprises one or more agents for specifically reducing expression of one or more short isoforms of PDE4D selected from PDE4D1, PDE4D2 and PDE4D6.

2. Composition for use according claim 1, wherein the subject has an International Society for Urological Pathology (ISUP) score of 2-5, preferably 4 or 5.

3. Composition for use according to claim 1 or 2, wherein the composition comprises one or more agents that allow for the expression in the prostate tumor of the subject selected from:
- an agent for expressing a dominant-negative form of PDE4D1 (Asp396Ala), PDE4D2 (Asp318Ala), and/or PDE4D6 (Asp329Ala);
- a proteolysis targeting chimera (PROTAC) agent specifically targeting PDE4D1, PDE4D2, and/or PDE4D6; and
- an RNA agent for specifically inhibiting the expression of PDE4D1, PDE4D2 and/or PDE4D6.

4. Composition for use according to any one of the preceding claims wherein the composition further comprises an agent that allows for the expression in the prostate tumor of the subject one or more of:
- an agent for expressing PDE4D4, PDE4D5 and/or PDE4D7 or a mutationally activated form thereof;
- A deubiquitinase-targeting chimera (DUBTAC) agent for specifically preventing the degradation of PDE4D4, PDE4D5, and/or PDE4D7.

5. Composition for use according to claim 3 or 4, wherein the PROTAC and/or DUBTAC specifically targets PDE4D1, PDE4D2, and/or PDE4D6, and/or PDE4D4, PDE4D5, and/or PDE4D7 with a PDE4D isotype specific targeting unit selected from: an antibody or antigen binding antibody fragment, a targeting peptide, a small molecule or an aptamer.

6. Composition for use according to claim 5, wherein the antibody or antibody fragment is selected from a single domain antibody (nanobody), a VHH fragment, a FAB fragment, a heavy chain antibody, a scFv antibody.

7. Composition for use according to any one of the preceding claims wherein the composition is administered prior to the antiandrogen, concurrent with the antiandrogen or after the antiandrogen.

8. Therapy for use according to any one of the preceding claims wherein the therapy further comprises locoregional therapy, preferably radical prostatectomy (RP) or radiation therapy after conclusion of the antiandrogen treatment, concurrent with the antiandrogen therapy or prior to antiandrogen therapy.

9. Composition for use according to any one of the preceding claims, wherein the antiandrogen is selected from abiraterone acetate, enzalutamide, apalutamide, or darolutamide, flutamide, bicalutamide, nilutamide and estrogens.

10. Composition for use according to any one of the preceding claims, wherein the antiandrogen is provided in a neoadjuvant, adjuvant or salvage setting.

11. Composition for use according to any one of the preceding claims wherein the antiandrogen is provided for 3 to 12 months, preferably between 4 and 10 months more preferably between 5 and 8 months.

12. An antiandrogen for use in the treatment, prevention or amelioration of prostate cancer in a subject in need thereof, wherein the subject is an intermediate to high-risk prostate cancer patients with localized disease
the use comprising administering to the subject the composition and an antiandrogen;
wherein the composition comprises one or more agents for specifically reducing expression of one or more short isoforms of PDE4D selected from PDE4D1, PDE4D2 and PDE4D6.

13. Antiandrogen for use according to claim 12, wherein the antiandrogen is selected from abiraterone acetate, enzalutamide, apalutamide, or darolutamide, flutamide, bicalutamide, nilutamide and estrogens.

14. Antiandrogen for use according to claim 12 or 13, wherein the antiandrogen is provided in a neoadjuvant, adjuvant or salvage setting.

15. Antiandrogen for use according to any one of claims 12 to 14 wherein the antiandrogen is provided for 3 to 12 months, preferably between 4 and 10 months more preferably between 5 and 8 months.
